# EUROPÄISCHE PATENTSCHRIFT

(11) **EP 1 077 703 B1**
(45) Veröffentlichungstag und Bekanntmachung des Hinweises auf die Patenterteilung: **26.03.2003**
(21) Anmeldenummer: 99923491.7
(22) Anmeldetag: 29.04.1999
(51) Int. Cl.: A61K 31/47, A61K 9/16, A61K 9/20, A61K 9/24

(54) **ORALE ARZNEIFORMEN MIT REPRODUZIERBARER WIRKSTOFFFREISETZUNG VON GATIFLOXACIN ODER PHARMAZEUTISCH VERWENDBAREN SALZEN ODER HYDRATEN**
ORAL MEDICINAL PREPARATIONS WITH REPRODUCIBLE RELEASE OF THE ACTIVE INGREDIENT GATIFLOXACIN OR ITS PHARMACEUTICALLY SUITABLE SALTS OR HYDRATES
FORMES GALENIQUES ORALES A LIBERATION REPRODUCTIBLE DU PRINCIPE ACTIF DE GATIFLOXACINE OU DE SELS ET D'HYDRATES UTILISABLES AU PLAN PHARMACEUTIQUE

(30) Priorität: 09.05.1998 DE 19820801
(43) Veröffentlichungstag der Anmeldung: 28.02.2001
(73) Patentinhaber: Grünenthal GmbH, 52078 Aachen (DE)
(72) Erfinder: BARTHOLOMÄUS, Johannes, D-52074 Aachen (DE); BETZING, Jürgen, D-53117 Bonn (DE)
(86) Internationale Anmeldenummer: EP9902893
(87) Internationale Veröffentlichungsnummer: WO99058129

(56) Entgegenhaltungen:
- EP-A- 0 230 295
- US-A- 5 436 253

## Beschreibung

Die Erfindung betrifft orale Arzneiformen mit reproduzierbarer Zerfallszeit und Wirkstofffreisetzung des Wirkstoff Gatifloxacin (1-cyclopropyl-6-fluoro-1,4-dihydro-8-methoxy-7-(3-methyl-1-piperazinyl)-4-oxo-3-chinolin-carbonsäure) oder pharmazeutisch verwendbaren Salzen oder Hydraten davon, sowie ein Verfahren zu deren Herstellung.

Formulierungen in Form fester Einzelarzneiformen wie z.B. Tabletten werden durch Verpressen hergestellt. Tabletten stellen die häufigste und wichtigste feste Arzneiform in der medizinischen Versorgung dar. Beispielhaft seien hier Sublingual-, Trink-, Buccal-, Brause-, Augentabletten, schnellfreisetzende Tabletten oder überzogene Tabletten aufgeführt. Diese werden aus fein kristallinen, gepulverten oder granulierten Arzneistoffen in der Regel unter Zusatz von Hilfsstoffen durch Pressen hergestellt.

Die physikalischen und chemischen Eigenschaften der einzelnen Wirkstoffe sind für die Herstellung von Tabletten entscheidend. Hierbei sind Dichte, Wasseranteil, Kristallform, Oberflächenstruktur, Teilchengröße, Löslichkeit, Fließeigenschaften, Hygroskopie und Qualitätsgrad des betreffenden Wirkstoffes zu nennen. Größeren Einfluß auf das Herstellungsverfahren pharmazeutisch hochwertiger Tabletten (D. Chulia, M. Deleuil; powder technology and pharmaceutical process, 1994) haben vor allen Wasseranteil, Teilchengröße, Kristallform und Löslichkeit der jeweiligen Wirkstoffe.

Die Herstellung von Tabletten erfolgt durch Verpressen von Pulvern oder Granulaten. Unter Granulieren wird das Überführen kleiner Pulverpartikel in größere Agglomerate bzw. Agglomeratkörner verstanden. Aus Granulaten hergestellte Tabletten besitzen in vielen Fällen größere mechanische Festigkeit als Pulverkomprimate. Dies wird durch die unebene und rauhe Oberfläche der Granulatkörner hervorgerufen, die größere Berührungsflächen besitzen, wodurch eine Erhöhung der Adhäsionskräfte hervorgerufen wird. Bei der Feuchtgranulation werden die Granulatkörner aus den Primärteilchen mit Hilfe einer Flüssigkeit hergestellt. Die Flüssigkeit, die aus der Gruppe von Wasser, Alkoholen oder polaren oder unpolaren Kohlenwasserstoffverbindungen ausgewählt werden kann, enthält in vielen Fällen meist noch sogenannte Bindemittel z. B. Polyvinylpolyvidon, vorgelatinierte Stärke oder Hydroxypropylcellulose.

Bestehen Tabletten aus Granulaten, so wird bei dieser Formulierungsart zwischen einer inneren und zumindest einer äußeren Phase unterschieden. In der inneren Phase befindet sich zumeist der Wirkstoff und andere Hilfsstoffe. Dieser Teil wird in einem Feucht- oder Trockenverfahren granuliert und als innere Phase des späteren Preßgutes bezeichnet. Im Feuchtgranulationsverfahren wird dem Substanzgemisch eine definierte Flüssigkeitsmenge zugeführt und granuliert. Hilfsstoffe aus der Gruppe der Bindemittel, Zerfallsförderer, Schmiermittel und/oder Füllstoffe sind die Inhaltsstoffe der sogenannten äußeren Phase. Beide Phasen werden gemischt und daran anschließend zu einer festen Arzneiform verpreßt.

Die Zerfallszeit von Tabletten und die damit verbundene Wirkstofffreisetzung ist ein wichtiges Indiz für die Bioverfügbarkeit im menschlichen Körper.

Der Zerfall von Tabletten ist eine Prüfmethode, um eine Aussage über eine wohl definierte Arzneiform treffen zu können. Zur Zerfallsbestimmung werden Tabletten in eine Apparatur eingebracht. Der Hauptteil der Apparatur besteht im allgemeinen aus einem starren Gestell mit Siebboden, das z. B. 6 zylindrische Prüfröhrchen aus Glas festgelegter Abmessungen enthält. Jedes Röhrchen kann mit einer Scheibe aus durchsichtigem Kunststoffmaterial oder vergleichbaren Materialien versehen sein, die bestimmte Bohrungen und V-förmige Einkerbungen haben. Die Prüfröhrchen werden senkrecht durch eine obere und eine untere Platte die aus Kunststoff bestehen kann, gehalten. An der Unterseite befindet sich ein Netz aus rostfreiem Stahldraht mit 2 mm Maschenweite. Die Apparatur wird durch einen Motor gleichmäßig 28-bis 32 mal je Minute auf- und abbewegt Die Apparatur wird in einem Gefäß aufgehängt, das eine geeignete Flüssigkeit enthält. Es sollte soviel Flüssigkeit in dem Gefäß vorhanden sein, daß das Drahtnetz am obersten Punkt seines Weges noch unter der Flüssigkeitsoberfläche eintaucht und am untersten Punkt noch vom Gefäßboden entfernt ist und die Öffnungen der Röhrchen über der Flüssigkeitsoberfläche bleiben. Die Flüssigkeit soll bei einer Temperatur von 36°C bis 38°C gehalten werden. Die Anforderungen sind erfüllt wenn nach einem definierten Zeitintervall der Zerfall erfolgt (Europäisches Arzneibuch, Ph. Eur.). Als flüssiges Medium können z. B. Wasser oder künstlicher Verdauungssaft dienen, die eine vorgegebenen Temperatur besitzen (Europäisches Arzneibuch, Ph. Eur.).

Wirkstofffreisetzungsuntersuchungen dienen zur Bestimmung der Auflösungsgeschwindigkeit von Wirkstoffen aus festen oralen Arzneistoffen wie Tabletten oder Kapseln, denn nur der gelöste Arzneistoff kann im Magen-Darm-Trakt resorbiert werden. Diese Untersuchungen erfolgen unter in vitro-Bedingungen z. B. in Wasser, künstlichem Magensaft mit einem pH von z. B. 1,2 oder künstlichem Darmsaft mit einem pH von z. B. 6,8 bei einer Temperatur von 37° C in einem definierten Zeitintervall (Europäisches Arzneibuch, Ph. Eur.). Es werden Blattrühreroder Drehkörbchen-Apparaturen verwendet. Beide Apparaturen bestehen aus einem Gefäß, einem Rührer und einem thermostatisiertem Bad. Das Gefäß ist mit einem Deckel in flanschförmiger Form bedeckt um das Verdampfen der Prüfflüssigkeit zu verhindern. Eine Öffnung zur Probeentnahme ist vorhanden, um die Konzentration des Arzneistoffes über die Zeit bestimmen zu können.

Formulierungen von festen Arzneiformen mit dem Wirkstoff Gatifloxacin oder pharmazeutisch verwendbaren Salzen oder Hydraten wurden bereits in der EP-B 0 230 295 beschrieben. Es hat sich gezeigt, daß reproduzierbare Zerfallszeiten und Wirkstofffreisetzungen der bekannten Tablettenformulierungen schwierig zu gewährleisten sind (siehe auch Tabelle 1 Beispiele 1 bis 6), da die Zerfallszeiten dieser festen Formulierungen in einem Bereich von 3 Minuten bis 600 Minuten variierten.

Die der vorliegenden Erfindung zugrundeliegende Aufgabe bestand darin, feste Arzneiformen, die reproduzierbare Zerfallszeiten und Wirkstofffreisetzungen besitzen und als Wirkstoff Gatifloxacin oder pharmazeutisch verwendbare Salze oder Hydrate davon und Hilfsstoffe enthalten, bereitzustellen sowie ein Verfahren zu deren Herstellung.

Es wurde gefunden, daß die an die Arzneiformen mit reproduzierbarer Zerfallszeit und Wirkstofffreisetzung gestellten Anforderungen, die als Wirkstoff Gatifloxacin (1-Cyclopropyl-6-fluoro-1,4-dihydro-8-methoxy-7-(3-methyl-1-piperazinyl)-4-oxo-3-chinolincarbonsäure) oder pharmazeutisch verwendbare Salze oder Hydrate davon enthalten, durch die erfindungsgemäße feste Arzneiform erfüllt wird, die mittels Granulation hergestellt wird und eine innere Phase und mindestens eine äußere Phase besitzt.

Gegenstand der Erfindung ist dementsprechend eine feste Arzneiform mit mehrphasigem Aufbau zur oralen Applikation, die aus einer inneren Phase mit dem Wirkstoff Gatifloxacin (1-Cyclopropyl-6-fluoro-1,4-dihydro-8-methoxy-7-(3-methyl-1-piperazinyl)-4-oxo-3-chinolincarbonsäure) oder pharmazeutisch verwendbaren Salzen oder Hydraten davon und Hilfsstoffen aus der Gruppe von Füllstoffen, Bindemitteln, Zerfallsförderern oder Mischungen davon und mindestens einer äußeren Phase bestehend aus zwingend mindestens einem Zerfallsförderer und weiteren Hilfsstoffen aus der Gruppe von mindestens einem Schmiermittel, gegebenenfalls Füllstoffen und/oder gegebenenfalls Bindemitteln, besteht.

Als feste Arzneiform seien beispielhaft Pellets, Kapseln, Tabletten oder Dragees erwähnt. Als bevorzugte feste Arzneiform werden Tabletten verwendet.

Die aus dem Wirkstoff Gatifloxacin oder pharmazeutisch verwendbaren Salzen oder Hydraten und Hilfsstoffen erhaltenen Granulate bilden die innere Phase. Die für die innere Phase verwendeten pharmazeutischen Hilfsstoffe stammen aus der Gruppe von Füllstoffen, Bindemitteln, Zerfallsförderern oder Mischungen davon.

Feste Formulierungen für den Wirkstoff Gatifloxacin oder pharmazeutisch verwendbare Salze oder Hydrate, die in einem Zeitintervall von 6 Minuten bis 30 Minuten vollständig zerfallen bei einer gleichzeitigen Wirkstofffreisetzung von über 80% innerhalb dieses Zeitraumes, sind nicht bekannt.

Der Anteil an Gatifloxacin oder pharmazeutisch verwendbaren Salzen oder Hydraten davon liegt im Bereich von 20 Gew.% bis 80 Gew.%, bezogen auf das Gesamtgewicht der oralen festen Arzneiform. Bevorzugt wird ein Bereich von 50 Gew.% bis 80 Gew.%, bezogen auf das Gesamtgewicht der oralen festen Arzneiform.

Unter dem Ausdruck "Füllstoffe" werden unter anderem Lactose, Stärke, Dicalciumphosphat, mikrokristalline Cellulose, Dextrose, Mannitol oder Mischungen davon verstanden.

Als Bindemittel können Hydroxypropylmethylcellulosen, Polyvinylpyrrolidine, Hydroxypropylcellulosen, Stärkekleister oder Mischungen davon eingesetzt werden.

Zur Gruppe der Zerfallsförderer gehören im Rahmen der vorliegenden Erfindung niedrig substituierte Hydroxypropylcellulosen, Crosspovidone, Crosscarmellose, Stärken, Pektine, Alginate, Tenside oder Mischungen davon. Besonders bevorzugt sind Cellulosen aus der Gruppe der mikrokristallinen Cellulosen, Hydroxypropylcellulosen, niedrig substituierter Hydroxypropylcellulosen oder Mischungen davon.

Als Bestandteil der äußeren Phase dienen zwingend mindestens ein Zerfallsförderer und weitere pharmazeutische Hilfsstoffe aus der Gruppe von mindestens einem Schmiermittel, gegebenenfalls Bindemitteln und/oder gegebenenfalls Füllstoffen.

Zur Gruppe der Schmiermittel, die in der äußeren Phase verwendet werden, seien beispielhaft Magnesiumstearat, Stearinsäure, Calciumstearat, Fettalkohole oder Mischungen davon aufgeführt. In der äußeren Phase werden zwingend neben mindestens einem Zerfallsförderer bevorzugt mindestens ein Schmiermittel, gegebenenfalls Bindemittel und/oder gegebenenfalls Füllstoffe eingesetzt.

Weiterhin ist ein Verfahren zur Herstellung fester mehrphasiger Arzneiformen zur oralen Applikation Erfindungsgegenstand, die als Wirkstoff Gatifloxacin oder pharmazeutisch verwendbare Salze oder Hydrate davon und Hilfsstoffe aus der Gruppe von Bindemitteln, Schmiermitteln, Füllstoffen und/oder Zerfallsförderern enthalten. Die innere Phase besteht aus Gatifloxacin, pharmazeutisch verwendbaren Salzen oder Hydraten davon und Hilfsstoffen aus der Gruppe von Bindemitteln, Füllstoffen und/oder Zerfallsförderern oder Mischungen davon, die in einem Mischwerkzeug aus der Gruppe von Mischern oder Knetern, bei einer Umdrehungszahl von 20 bis 500 UpM (Umdrehungszahl pro Minute) in Gegenwart eines Granulierflüssigkeitanteils von 20 Gew.% bis 80 Gew.%, bezogen auf die eingesetzte Gesamtmenge an Komponenten der inneren Phase innerhalb von 0,5 Minuten bis 20 Minuten in das Granulat überführt wird. Hierauf wird das Granulat getrocknet, gesiebt und mit Hilfsstoffen für mindestens eine äußere Phase bestehend aus zwingend mindestens einem Zerfallsförderer und weiteren Hilfsstoffen aus der Gruppe von mindestens einem Schmiermittel, gegebenenfalls Bindemitteln und/oder gegebenenfalls Füllstoffen, die nicht in der inneren Phase mitgranuliert worden sind, vermischt, und in eine feste Arzneiform überführt. Als feste Arzneiform wird die Tablette bevorzugt.

Bevorzugt wird ein Verfahren, bei dem für die innere Phase der festen Arzneiform, innerhalb von 0,5 Minuten bis 10 Minuten, ein Granulierflüssigkeitanteil von 20 Gew.% bis 70 Gew.% bezogen auf die eingesetzte Gesamtmenge der Komponenten der inneren Phase und eine Umdrehungszahl des Mischers von 20 bis 450 UpM verwendet wird.

Besonders bevorzugt ist ein Verfahren bei dem für die innere Phase der festen Arzneiform, innerhalb von 1 Minute bis 7 Minuten, ein Granulierflüssigkeitsanteil von 20 Gew.% bis 60 Gew.% bezogen auf die eingesetzte Gesamtmenge der Komponenten der inneren Phase und eine Umdrehungszahl des Mischers von 20 bis 400 UpM verwendet wird.

Die gemäß dem erfindungsgemäßen Verfahren aus Granulaten hergestellten Tabletten zeichnen sich dadurch aus, daß sie mindestens zu 3 Gew.% und maximal zu 20 Gew.% aus einer oder mehreren äußeren Phasen bestehen, die mindestens ein Schmiermittel, mindestens einen Zerfallsförderer, gegebenenfalls Bindemittel und/oder gegebenenfalls Füllstoffe, enthalten. Bevorzugt wird ein Anteil, der oberhalb von 5 Gew.% liegt.

Die für das Verfahren verwendeten Mischwerkzeuge gehören zur Gruppe der Mischer und Kneter. Beispielhaft seien Flugscharmischer oder kleinere Mischer und Kneter von Lödige, Niro-Fielder oder Baker-Perkins erwähnt.

Die Zerfallszeiten der Referenzbeispiele 1 bis 6 variieren in einem Bereich von 3 Minuten bis 10 Stunden, wie aus der Tabelle 1 zu ersehen ist. Die erfindungsgemäße Arzneiform, gemäß den Beispielen 7 bis 14, gemäß Tabelle 2, zeigt demgegenüber ein Zerfallsintervall von 6,5 Minuten bis 25 Minuten, wodurch die Reproduzierbarkeit und Therapiesicherheit gewährleistet werden kann.

**Tabelle 1.**

| Beispiel | Härte [N] | Zerfallszeit der Preßlinge [min.] |
|---|---|---|
| 1 | 140-150 | ∼600,0 |
| 2 | 140-150 | 3,0 |
| 3 | 140-150 | 180,0 |
| 4 | 140-150 | 6,0 |
| 5 | 140-150 | 150,0 |
| 6 | 140-150 | 146,0 |

Die Freisetzungskurven der Beispiele 1 bis 4 verlaufen mit sehr unterschiedlichen Freisetzungsprofilen des Wirkstoffs. Bei den Freisetzungskurven der Beispiele 1 und 3 ist innerhalb der ersten Stunde nur circa 50 % des Wirkstoffs verfügbar. Die Beispiele 2 und 4 weisen demgegenüber eine vollständige Wirkstofffreisetzung trotz gleicher Formulierung und gleicher Bestandteile der Preßlinge auf. Die Reproduzierbarkeit der Freisetzung des Wirkstoffes ist aus den bekannten Zusammensetzungen nicht gewährleistet, so daß es somit zu einem niedrigen Freisetzungsgrad im Körper über einen unerwünscht langen Zeitraum, kommen kann.

**Tabelle 2**

| **Beispiel** | **Härte [N]** | **Zerfallszeit der Preßlinge [min.]** |
|---|---|---|
| 7 | 140-150 | 6,5 |
| 8 | 140-150 | 8,8 |
| 9 | 140-150 | 7,5 |
| 10 | 140-150 | 16,5 |
| 11 | 140-150 | 9,5 |
| 12 | 140-150 | 18,5 |
| 13 | 140-150 | 20,5 |
| 14 | 140-150 | 25,0 |

Die erfindungsgemäßen Arzneiformen der Beispiele 7 bis 14 zerfallen vollständig in einem Zeitintervall von 6,5 Minuten bis 25 Minuten. Bei Betrachtung des Freisetzungsprofils wurde innerhalb der ersten 30 Minuten eine Wirkstofffreisetzung von mindestens 80 % beobachtet und eine vollständige Freisetzung des Wirktoffes nach 60 Minuten.

Mit der erfindungsgemäßen festen Arzneiform wird die pharmazeutisch-technologische Qualität für die bis dahin nicht reproduzierbaren Zerfallszeiten und Wirkstofffreisetzungen sowie die damit verbundene Therapiesicherheit gewährleistet.

### Beispiele

Die Granulation erfolgte in einem Lödige Schnellmischer FM5 und die Tablettenherstellung mit einer Fette Exzenterpresse. Die Ansatzgrößen betrugen jeweils 800 g. Der Ausdruck "min." bedeutet Minuten.
Der Ausdruck "UpM" bedeutet Umdrehungen pro Minute.

### Beispiel 1

Man siebte 110,47 g mikrokristalline Cellulose und 81 g L-HPC (Hydroxypropylcellulose) durch ein 0,8 mm Sieb und mischte anschließend beide Substanzen mit 586,13 g Gatifloxacin (Wassergehalt 7,87 Gew.%) im Schnellmischer 5 min. Die Mischung wurde mit 700 ml einer wäßrigen Hydroxypropylcelluloselösung 5 min. bei einer Umdrehungszahl des Mischflügels von 385 UpM granuliert. Das feuchte Granulat wurde anschließend durch ein 3 mm Sieb gesiebt und bei 50° C ca. 17 Stunden getrocknet. Nach erneutem Sieben des getrockneten Granulats erfolgte die Zugabe von 16,20 g Magnesiumstearat und die Verpressung zu Tabletten mit einer Härte von 140 bis 150 N.

### Beispiel 2

Man siebte 110,47 g mikrokristalline Cellulose und 81 g L-HPC durch ein 0,8 mm Sieb und mischte anschließend beide Substanzen mit 586,13 g Gatifloxacin (Wassergehalt 7,87 Gew.%) im Schnellmischer 5 min. Die Mischung wurde mit 500 ml einer wäßrigen Hydroxypropylcelluloselösung 1 min. bei einer Umdrehungszahl des Mischflügels von 215 UpM granuliert. Das feuchte Granulat wurde anschließend durch ein 3 mm Sieb gesiebt und bei 50° C ca. 17 Stunden getrocknet. Nach erneutem Sieben des getrockneten Granulats erfolgte die Zugabe von 16,20 g Magnesiumstearat und die Verpressung zu Tabletten mit einer Härte von 140 bis 150 N.

### Beispiel 3

Man siebte 110,47 g mikrokristalline Cellulose und 81 g L-HPC durch ein 0,8 mm Sieb und mischte anschließend beide Substanzen mit 586,13 g Gatifloxacin (Wassergehalt 7,87 Gew.%) im Schnellmischer 5 min. Die Mischung wurde mit 700 ml einer wäßrigen Hydroxypropylcelluloselösung 5 min. bei einer Umdrehungszahl des Mischflügels von 215 UpM granuliert. Das feuchte Granulat wurde anschließend durch ein 3 mm Sieb gesiebt und bei 50° C ca. 17 Stunden getrocknet. Nach erneutem Sieben des getrockneten Granulats erfolgte die Zugabe von 16,20 g Magnesiumstearat und die Verpressung zu Tabletten mit einer Härte von 140 bis 150 N.

### Beispiel 4

Man siebte 110,47 g mikrokristalline Cellulose und 81 g L-HPC durch ein 0,8 mm Sieb und mischte anschließend beide Substanzen mit 586,13 g Gatifloxacin (Wassergehalt 7,87 Gew.%) im Schnellmischer 5 min. Die Mischung wurde mit 500 ml einer wäßrigen Hydroxypropylcelluloselösung 1 min. bei einer Umdrehungszahl des Mischflügels von 385 UpM granuliert. Das feuchte Granulat wurde anschließend durch ein 3 mm Sieb gesiebt und bei 50° C für ca. 17 Stunden getrocknet. Nach erneutem Sieben des getrockneten Granulats erfolgte die Zugabe von 16,20 g Magnesiumstearat und die Verpressung zu Tabletten mit einer Härte von 140 bis 150 N.

### Beispiel 5

Man siebte 110,47 g mikrokristalline Cellulose und 81 g L-HPC durch ein 0,8 mm Sieb und mischte anschließend beide Substanzen mit 586,13 g Gatifloxacin (Wassergehalt 7,87 Gew.%) im Schnellmischer 5 min. Die Mischung wurde mit 500 ml einer wäßrigen Hydroxypropylcelluloselösung 5 min. bei einer Umdrehungszahl des Mischflügels von 385 UpM granuliert. Das feuchte Granulat wurde anschließend durch ein 3 mm Sieb gesiebt und bei 50° C für ca. 17 Stunden getrocknet. Nach erneutem Sieben des getrockneten Granulats erfolgte die Zugabe von 16,20 g Magnesiumstearat und die Verpressung zu Tabletten mit einer Härte von 140 bis 150 N.

### Beispiel 6

Man siebte 110,47 g mikrokristalline Cellulose und 81 g L-HPC durch ein 0,8 mm Sieb und mischte anschließend beide Substanzen mit 586,13 g Gatifloxacin (Wassergehalt 7,87 Gew.%) im Schnellmischer 5 min. Die Mischung wurde mit 500 ml einer wäßrigen Hydroxypropylcelluloselösung 1 min. bei einer Umdrehungszahl des Mischflügels von 215 UpM granuliert. Das feuchte Granulat wurde anschließend durch ein 3 mm Sieb gesiebt und bei 50° C für ca. 17 Stunden getrocknet. Nach erneutem Sieben des getrockneten Granulats erfolgte die Zugabe von 16,20 g Magnesiumstearat und die Verpressung zu Tabletten mit einer Härte von 140 bis 150 N.

Für die nachfolgenden Beispiele 7 bis 14 weisen die Tabletten eine erfindungsgemäße Zusammensetzung auf.

### Beispiel 7

102,668 g mikrokristalline Cellulose und 40,50 g L-HPC wurden durch ein 0,8 mm Sieb gesiebt und mit 582,53 g Gatifloxacin (Wassergehalt 7,30 Gew.%) in einem Schnellmischer 5 min. gemischt. Die Mischung wurde mit 300 ml einer wäßrigen Hydroxypropylcelluloselösung (16,20 g) 1 min. bei einer Umdrehungszahl des Mischflügels von 215 UpM granuliert. Das feuchte Granulat wurde anschließend durch ein 3 mm Sieb gesiebt und bei 50° C ca. 17 Stunden getrocknet. Nach erneutem Sieben des getrockneten Granulats erfolgte die Zugabe von 11,408 g mikrokristalliner Cellulose, 40,50 g L-HPC und 16,20 g Magnesiumstearat. Das Granulat wurde zu Tabletten mit einer Härte von 140 bis 150 N verpreßt.

### Beispiel 8

102,668 g mikrokristalline Cellulose und 40,50 g L-HPC wurden durch ein 0,8 mm Sieb gesiebt und mit 582,53 g Gatifloxacin (Wassergehalt 7,30 Gew.%) in einem Schnellmischer 5 min. gemischt. Die Mischung wurde mit 400 ml einer wäßrigen Hydroxypropylcelluloselösung (16,20 g) 1 min. bei einer Umdrehungszahl des Mischflügels von 215 UpM granuliert, das feuchte Granulat durch ein 3 mm Sieb gesiebt und bei 50° C ca. 17 Stunden getrocknet. Nach erneutem Sieben des getrockneten Granulats erfolgte die Zugabe von 11,408 g mikrokristalliner Cellulose, 40,50 g L-HPC und 16,20 g Magnesiumstearat. Das Granulat wurde zu Tabletten mit einer Härte von 140 bis 150 N verpreßt.

### Beispiel 9

102,668 g mikrokristalline Cellulose und 40,50 g L-HPC wurden durch ein 0,8 mm Sieb gesiebt und mit 582,53 g Gatifloxacin (Wassergehalt 7,30 Gew.%) in einem Schnellmischer 5 min. gemischt. Die Mischung wurde mit 300 ml einer wäßrigen Hydroxypropylcelluloselösung (16,20 g) 5 min. bei einer Umdrehungszahl des Mischflügels von 215 UpM granuliert. Das feuchte Granulat wurde anschließend durch ein 3 mm Sieb gesiebt und bei 50° C ca. 17 Stunden getrocknet. Nach erneutem Sieben des getrockneten Granulats erfolgte die Zugabe von 11,408 g mikrokristalliner Cellulose, 40,50 g L-HPC und 16,20 g Magnesiumstearat. Das Granulat wurde zu Tabletten mit einer Härte von 140 bis 150 N verpreßt.

### Beispiel 10

102,668 g mikrokristalline Cellulose und 40,50 g L-HPC wurden durch ein 0,8 mm Sieb gesiebt und mit 582,53 g Gatifloxacin (Wassergehalt 7,30 Gew.%) in einem Schnellmischer 5 min. gemischt. Die Mischung wurde mit 400 ml einer wäßrigen Hydroxypropylcelluloselösung (16,20 g) 5 min. bei einer Umdrehungszahl des Mischflügels von 215 UpM granuliert, das feuchte Granulat durch ein 3 mm Sieb gesiebt und bei 50° C ca. 17 Stunden getrocknet. Nach erneutem Sieben des getrockneten Granulats erfolgte die Zugabe von 11,408 g mikrokristalliner Cellulose, 40,50 g L-HPC und 16,20 g Magnesiumstearat. Das Granulat wurde zu Tabletten mit einer Härte von 140 bis 150 N verpreßt.

### Beispiel 11

102,668 g mikrokristalline Cellulose und 40,50 g L-HPC wurden durch ein 0,8 mm Sieb gesiebt und mit 582,53 g Gatifloxacin (Wassergehalt 7,30 Gew.%) in einem Schnellmischer 5 min. gemischt. Die Mischung wurde mit 300 ml einer wäßrigen Hydroxypropylcelluloselösung (16,20 g) 1 min. bei einer Umdrehungszahl des Mischflügels von 385 UpM granuliert. Das feuchte Granulat wurde anschließend durch ein 3 mm Sieb gesiebt und bei 50° C ca. 17 Stunden getrocknet. Nach erneutem Sieben des getrockneten Granulats erfolgte die Zugabe von 11,408 g mikrokristalliner Cellulose, 40,50 g L-HPC und 16,20 g Magnesiumstearat. Das Granulat wurde zu Tabletten mit einer Härte von 140 bis 150 N verpreßt.

### Beispiel 12

102,668 g mikrokristalline Cellulose und 40,50 g L-HPC wurden durch ein 0,8 mm Sieb gesiebt und mit 582,53 g Gatifloxacin (Wassergehalt 7,30 Gew.%) in einem Schnellmischer 5 min. gemischt. Die Mischung wurde mit 400 ml einer wäßrigen Hydroxypropylcelluloselösung (16,20 g) 1 min. bei einer Umdrehungszahl des Mischflügels von 385 UpM granuliert. Das feuchte Granulat wurde anschließend durch ein 3 mm Sieb gesiebt und bei 50° C ca. 17 Stunden getrocknet. Nach erneutem Sieben des getrockneten Granulats erfolgte die Zugabe von 11,408 g mikrokristalliner Cellulose, 40,50 g L-HPC und 16,20 g Magnesiumstearat. Das Granulat wurde zu Tabletten mit einer Härte von 140 bis 150 N verpreßt.

### Beispiel 13

102,668 g mikrokristalline Cellulose und 40,50 g L-HPC wurden durch ein 0,8 mm Sieb gesiebt und mit 582,53 g Gatifloxacin (Wassergehalt 7,30 Gew.%) in einem Schnellmischer 5 min. gemischt. Die Mischung wurde mit 300 ml einer wäßrigen Hydroxypropylcelluloselösung (16,20 g) 5 min. bei einer Umdrehungszahl des Mischflügels von 385 UpM granuliert. Das feuchte Granulat wurde anschließend durch ein 3 mm Sieb gesiebt und bei 50° C ca. 17 Stunden getrocknet. Nach erneutem Sieben des getrockneten Granulats erfolgte die Zugabe von 11,408 g mikrokristalliner Cellulose, 40,50 g L-HPC und 16,20 g Magnesiumstearat. Das Granulat wurde zu Tabletten mit einer Härte von 140 bis 150 N verpreßt.

### Beispiel 14

102,668 g mikrokristalline Cellulose und 40,50 g L-HPC wurden durch ein 0,8 mm Sieb gesiebt und mit 582,53 g Gatifloxacin (Wassergehalt 7,30 Gew.%) in einem Schnellmischer 5 min. gemischt. Die Mischung wurde mit 400 ml einer wäßrigen Hydroxypropylcelluloselösung (16,20 g) 5 min. bei einer Umdrehungszahl des Mischflügels von 385 UpM granuliert. Das feuchte Granulat wurde anschließend durch ein 3 mm Sieb gesiebt und bei 50° C ca. 17 Stunden getrocknet. Nach erneutem Sieben des getrockneten Granulats erfolgte die Zugabe von 11,408 g mikrokristalliner Cellulose, 40,50 g L-HPC und 16,20 g Magnesiumstearat. Das Granulat wurde zu Tabletten mit einer Härte von 140 bis 150 N verpreßt.

## Patentansprüche

1. Feste Arzneiform mit mehrphasigem Aufbau zur oralen Applikation, enthaltend Gatifloxacin oder pharmazeutisch verwendbare Salze oder Hydrate davon und Hilfsstoffe aus der Gruppe von Füllstoffen, Bindemitteln, Schmiermitteln, Zerfallsförderern oder Mischungen davon, **dadurch gekennzeichnet, daß** die innere Phase den Wirkstoff Gatifloxacin oder pharmazeutisch verwendbare Salze oder Hydrate davon, zusammen mit Hilfsstoffen aus der Gruppe von Bindemitteln, Füllstoffen, Zerfallsförderern oder Mischungen davon, enthält und mindestens eine äußere Phase bestehend aus zwingend mindestens einem Zerfallsförderer und weiteren Hilfsstoffen aus der Gruppe von mindestens einem Schmiermittel, gegebenenfalls Füllstoffen und/oder gegebenenfalls Bindemitteln enthält.

2. Arzneiform nach Anspruch 1, **dadurch gekennzeichnet, daß** der Anteil an Gatifloxacin oder pharmazeutisch verwendbaren Salzen oder Hydraten davon im Bereich von 20 Gew.% bis 80 Gew.%, bezogen auf das Gesamtgewicht der oralen festen Arzneiform, liegt.

3. Arzneiform nach Anspruch 1, **dadurch gekennzeichnet, daß** der Anteil an Gatifloxacin oder pharmazeutisch verwendbaren Salzen oder Hydraten davon, bevorzugt in einem Bereich von 50 Gew.% bis 80 Gew.% bezogen auf das Gesamtgewicht der oralen festen Arzneiform, liegt.

4. Arzneiform nach Anspruch 1, **dadurch gekennzeichnet, daß** der Anteil einer oder mehrerer äußerer Phasen am Gesamtgewicht der festen, oralen Arzneiform mindestens 3 Gew.% beträgt.

5. Arzneiform nach Anspruch 1, **dadurch gekennzeichnet, daß** Füllstoffe aus der Gruppe von Lactose, Stärke, Dicalciumphosphat, mikrokristalline Cellulose, Mannitol, Dextrose oder Mischungen davon enthalten sind.

6. Arzneiform nach Anspruch 1, **dadurch gekennzeichnet, daß** Bindemittel aus der Gruppe von Hydroxypropylmethylcellulosen, Polyvinylpyrrolidine, Hydroxypropylcellulosen, Stärkekleister oder Mischungen davon enthalten sind.

7. Arzneiform nach Anspruch 1, **dadurch gekennzeichnet, daß** zerfallsfördernde Substanzen aus der Gruppe von niedrig substituierten Hydroxypropylcellulosen, Crosspovidonen, Crosscarmellosen, Stärken, Pektinen, Alginaten, Tensiden oder Mischungen davon enthalten sind.

8. Arzneiform nach Anspruch 1, **dadurch gekennzeichnet, daß** Schmiermittel aus der Gruppe von Magnesiumstearat, Stearinsäure, Calciumstearat, Fettalkohole oder Mischungen davon enthalten sind.

9. Verfahren zur Herstellung einer festen Arzneiform mit mehrphasigem Aufbau zur oralen Applikation, enthaltend Gatifloxacin oder pharmazeutisch verwendbare Salze oder Hydrate davon und Hilfsstoffe aus der Gruppe von Bindemitteln, Schmiermitteln, Füllstoffen, Zerfallsförderern oder Mischungen davon, **dadurch gekennzeichnet, daß** man die innere Phase enthaltend Gatifloxacin oder pharmazeutisch verwendbare Salze oder Hydrate davon mit Hilfsstoffen aus der Gruppe von Füllstoffen, Bindemitteln, Zerfallsförderern oder Mischungen davon und einem Granulierflüssigkeitanteil von 20 Gew.% bis 80 Gew.%, bezogen auf die eingesetzte Gesamtmenge an Komponenten der inneren Phase, innerhalb von 0,5 Minuten bis 20 Minuten in das Granulat überführt, trocknet, siebt und die erhaltene innere Phase zwingend mit mindestens einem Zerfallsförderer und weiteren Hilfsstoffen aus der Gruppe von mindestens einem Schmiermittel, gegebenenfalls Bindemitteln und/oder gegebenenfalls Füllstoffen, für mindestens eine äußere Phase vermischt, und in eine feste Arzneiform überführt.

10. Verfahren nach Anspruch 9, **dadurch gekennzeichnet, daß** die innere Phase der festen Arzneiform bevorzugt innerhalb von 0,5 Minuten bis 10 Minuten mit einem Granulierflüssigkeitanteil von 20 Gew.% bis 70 Gew.% bezogen auf die eingesetzte Gesamtmenge der Komponenten der inneren Phase hergestellt wird.

11. Verfahren nach Anspruch 9 bis 10, **dadurch gekennzeichnet, daß** die innere Phase der festen Arzneiform innerhalb von 1 Minute bis 7 Minuten mit einem Granulierflüssigkeitanteil von 20 Gew.% bis 60 Gew.% bezogen auf die eingesetzte Gesamtmenge der Komponenten der inneren Phase hergestellt wird.

## Claims

1. Solid medicinal form of multiphase structure for oral administration, containing gatifloxacin or pharmaceutically acceptable salts or hydrates thereof and auxiliary substances from the group comprising fillers, binders, lubricants, disintegration aids or mixtures thereof, **characterized in that** the inner phase contains the active ingredient gatifloxacin or pharmaceutically acceptable salts or hydrates thereof, together with auxiliary substances from the group comprising binders, fillers, disintegration aids or mixtures thereof, and at least one outer phase necessarily consisting of at least one disintegration aid and further auxiliary substances from the group comprising at least one lubricant, optionally fillers and/or optionally binders.

2. Medicinal form according to Claim 1, **characterized in that** the proportion of gatifloxacin or pharmaceutically acceptable salts or hydrates thereof is in the range 20 wt.% to 80 wt.%, based on the total weight of the solid oral medicinal form.

3. Medicinal form according to Claim 1, **characterized in that** the proportion of gatifloxacin or pharmaceutically acceptable salts or hydrates thereof is preferably in a range 50 wt.% to 80 wt.%, based on the total weight of the solid oral medicinal form.

4. Medicinal form according to Claim 1, **characterized in that** the proportion of one or more outer phases is at least 3 wt.%, based on the total weight of the solid oral medicinal form.

5. Medicinal form according to Claim 1, **characterized in that** it contains fillers from the group comprising lactose, starch, dicalcium phosphate, microcrystalline cellulose, mannitol, dextrose or mixtures thereof.

6. Medicinal form according to Claim 1, **characterized in that** it contains binders from the group comprising hydroxypropyl methyl celluloses, polyvinylpyrrolidones, hydroxypropyl celluloses, starch mucilage or mixtures thereof.

7. Medicinal form according to Claim 1, **characterized in that** it contains disintegration aids from the group comprising hydroxypropyl celluloses with a low degree of substitution, crosspovidones, crosscarmelloses, starches, pectins, alginates, surfactants or mixtures thereof.

8. Medicinal form according to Claim 1, **characterized in that** it contains lubricants from the group comprising magnesium stearate, stearic acid, calcium stearate, fatty alcohols or mixtures thereof.

9. Process for the preparation of a solid medicinal form of multiphase structure for oral administration, containing gatifloxacin or pharmaceutically acceptable salts or hydrates thereof and auxiliary substances from the group comprising binders, lubricants, fillers, disintegration aids or mixtures thereof, **characterized in that** the inner phase, containing gatifloxacin or pharmaceutically acceptable salts or hydrates thereof with auxiliary substances from the group comprising fillers, binders, disintegration aids or mixtures thereof, and with a proportion of 20 wt.% to 80 wt.% of granulating liquid, based on the total amount of inner phase components used, is converted to granules over 0.5 minute to 20 minutes, the granules are dried and sieved and the resulting inner phase is necessarily mixed with at least one disintegration aid and further auxiliary substances from the group comprising at least one lubricant, optionally binders and/or optionally fillers, for at least one outer phase, and converted to a solid medicinal form.

10. Process according to Claim 9, **characterized in that** the inner phase of the solid medicinal form is preferably prepared over 0.5 minute to 10 minutes with a proportion of 20 wt.% to 70 wt.% of granulating liquid, based on the total amount of inner phase components used.

11. Process according to Claims 9 and 10, **characterized in that** the inner phase of the solid medicinal form is prepared over 1 minute to 7 minutes with a proportion of 20 wt.% to 60 wt.% of granulating liquid, based on the total amount of inner phase components used.

## Revendications

1. Médicament solide à structure comprenant plusieurs phases, destiné à être administré par voie orale, contenant de la gatifloxacine ou ses sels ou hydrates acceptables du point de vue pharmaceutique et des substances auxiliaires du groupe de charges, de liants, de lubrifiants, d'accélérateurs de désintégration ou de mélanges de ces substances, **caractérisé en ce que** la phase interne contient comme substance active la gatifloxacine ou ses sels ou hydrates acceptables du point de vue pharmaceutique, conjointement avec des substances auxiliaires du groupe de liants, de charges, d'accélérateurs de désintégration ou de mélanges de ces substances, et **en ce qu'**il contient au moins une phase externe constituée obligatoirement d'au moins un accélérateur de désintégration et d'autres substances auxiliaires du groupe d'au moins un lubrifiant, éventuellement de charges, et/ou le cas échéant de liants.

2. Médicament suivant la revendication 1, **caractérisé en ce que** la proportion de gatifloxacine ou de ses sels ou hydrates acceptables du point de vue pharmaceutique se situe dans la plage de 20 % en poids à 80 % en poids, par rapport au poids total du médicament sous la forme orale solide.

3. Médicament suivant la revendication 1, **caractérisé en ce que** la proportion de gatifloxacine ou de ses sels ou hydrates acceptables du point de vue pharmaceutique se situe avantageusement dans une plage de 50 à 80 % en poids, par rapport au poids total du médicament sous la forme orale solide.

4. Médicament suivant la revendication 1, **caractérisé en ce que** la proportion d'une ou plusieurs phases externes relativement au poids total du médicament sous la forme orale solide s'élève à au moins 3 % en poids.

5. Médicament suivant la revendication 1, **caractérisé en ce qu'**il contient des charges du groupe du lactose, de l'amidon, du phosphate dicalcique, de la cellulose microcristalline, du mannitol, du dextrose ou de mélanges de ces charges.

6. Médicament suivant la revendication 1, **caractérisé en ce qu'**il contient des liants du groupe des hydroxy-propylméthylcelluloses, des polyvinylpyrrolidines, des hydroxypropylcelluloses, des empois d'amidon ou de mélanges de ces liants.

7. Médicament suivant la revendication 1, **caractérisé en ce qu'**il contient des substances accélérant la désintégration choisies dans le groupe d'hydroxy-propylcelluloses faiblement substituées, de crospovidones, de croscarmelloses, de substances amylacées, de pectines, d'alginates, d'agents tensio-actifs ou de mélanges de ces substances.

8. Médicament suivant la revendication 1, **caractérisé en ce qu'**il contient des lubrifiants du groupe du stéarate de magnésium, de l'acide stéarique, du stéarate de calcium, d'alcools gras ou de leurs mélanges.

9. Procédé de préparation d'un médicament sous forme solide à structure comprenant plusieurs phases, destiné à l'administration orale, contenant de la gatifloxacine ou ses sels ou hydrates acceptables du point de vue pharmaceutique et des substances auxiliaires du groupe de liants, de lubrifiants, de charges, d'accélérateurs de désintégration ou de mélanges de ces substances, **caractérisé en ce qu'**on transforme en une période de 0,5 à 20 minutes en produit granulé la phase interne contenant la gatifloxacine ou ses sels ou hydrates acceptables du point de vue pharmaceutique avec des substances auxiliaires du groupe de charges, de liants, d'accélérateurs de désintégration ou leurs mélanges et avec une proportion de liquide de granulation de 20 à 80 % en poids par rapport à la quantité totale utilisée de composants de la phase interne, on sèche, on tamise et on mélange la phase interne obtenue obligatoirement avec au moins un accélérateur de désintégration et d'autres substances auxiliaires du groupe d'au moins un lubrifiant, éventuellement des liants et/ou le cas échéant des charges, pour former au minimum une phase externe, et on fait prendre au mélange une forme médicamenteuse solide.

10. Procédé suivant la revendication 9, **caractérisé en ce que** la phase interne du médicament sous la forme solide est avantageusement produite dans un intervalle de 0,5 à 10 minutes avec une proportion de liquide de granulation de 20 à 70 % en poids par rapport à la quantité totale utilisée des composants de la phase interne.

11. Procédé suivant les revendications 9 et 10, **caractérisé en ce qu'**on prépare la phase interne de la forme médicamenteuse solide en une période de 1 à 7 minutes avec une proportion de liquide de granulation de 20 à 60 % en poids par rapport à la quantité totale utilisée des composants de la phase interne.
